(19) Europäisches Patentamt
European Patent Office
Office européen des brevets

(11) Publication number: **0 039 111**
**B1**

(12) # EUROPEAN PATENT SPECIFICATION

(45) Date of publication of patent specification: **24.08.83**

(21) Application number: **81200421.6**

(22) Date of filing: **13.04.81**

(51) Int. Cl.³: **C 07 C 59/125**, C 07 C 51/285, B 01 J 23/44

(54) **A process for the preparation of alkoxyalkanoic acids.**

(30) Priority: **29.04.80 US 144807**

(43) Date of publication of application:
**04.11.81 Bulletin 81/44**

(45) Publication of the grant of the patent:
**24.08.83 Bulletin 83/34**

(84) Designated Contracting States:
**BE DE FR GB IT NL**

(56) References cited:
**EP - A - 0 018 681**
**GB - A - 956 100**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Slaugh, Lynn Henry**
**610 Winter Oaks**
**Houston Texas 77079 (US)**
Inventor: **Willis, Carl Lesley**
**15922 Red Willow**
**Houston Texas 77084 (US)**

(74) Representative: **Puister, Antonius Tonnis, Mr. et al,**
**P.O. Box 302**
**NL-2501 CH The Hague (NL)**

**EP 0 039 111 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

A process for the preparation of alkoxyalkanoic acids

The invention relates to a process for the preparation of an alkoxyalkanoic acid of the general formula:

$$RO(CH_2CHR^1O)_nCH_2CO_2H \quad (I)$$

wherein R represents an alkyl group, $R^1$ represents a hydrogen atom or a methyl group or — on the individual molecule — a mixture thereof and n is an integer, by reacting an alkoxyalkanol of the general formula:

$$RO(CH_2CHR^1O)_nCH_2CH_2OH \quad (II)$$

wherein R, $R^1$ and n have the same meaning as in formula I, in the liquid phase with an oxidation agent in the presence of an alkali metal hydroxide and a catalytically effective amount of a noble metal of Group VIII of the Periodic Table of the Elements.

Alkoxyalkanoic acids find use as anionic detergents. These acids being composed of only the elements C, H and O do not pose the environmental problems that other detergents containing hetero-atoms such as N, S, and P pose. Commercially, the alkoxyalkanoic acids are prepared in a two-step process of first reacting an alkoxyalkanol with sodium and then reacting the resultant ethoxide with the sodium salt of chloroacetic acid. A one-step process avoiding the use of chloroacetic acid and providing a NaCl-free product would be of significant commercial interest.

Japanese Patent Publication No. 50—96516 discloses a process for the preparation of carboxylic acid salts by liquid phase dehydrogenative oxidation of alcohols with an alkali metal hydroxide in the presence of precious metal catalysts, including palladium and platinum. This process uses a relatively high temperature, 100—270°C. These high temperatures can degrade the ether linkages, especially in the highly ethoxylated alcohols of formula II.

In the process described in the beginning of this specification, and as described in U.S. patent specification 3,342,858, the oxidation agent is an oxygen-containing gas used at a temperature between 20 and 75°C in the presence of a platinum catalyst. This known process presents all the problems attendant with gas-liquid phase operations, such as gaseous diffusion limitations, additional equipment, such as compressors needed to process the gas phase.

A process for converting the alkoxyalkanols of formula II into the corresponding alkoxyalkanoic acids of formula I has now been found, in which a special combination of oxidation agent and noble metal catalyst is applied. This special combination overcomes many of the problems of gas-liquid phase operations and provides an extremely mild, low temperature process. It minimizes degradation products, provides high conversions of the alkoxyalkanol and the oxidation agent, with high selectivity to the alkali metal salt of the alkoxyalkanoic acid. The process is particularly suited to the detergent range ethoxylate or propoxylate alcohols.

Accordingly, the invention provides a process for the preparation of an alkoxyalkanoic acid of the general formula:

$$RO(CH_2CHR^1O)_nCH_2CO_2H \quad (I)$$

wherein R represents an alkyl group, optionally substituted by $—OR^2$, COOH, $CONH_2$, $COOR^2$, wherein $R^2$ represents an alkyl or aryl group, $R^1$ represents a hydrogen atom or a methyl group or — on the individual molecule — a mixture thereof and n is an integer, by reacting an alkoxyalkanol of the general formula:

$$RO(CH_2CHR^1O)_nCH_2CH_2OH \quad (II)$$

wherein R, $R^1$ and n have the same meaning as in formula I, in the liquid phase with an oxidation agent in the presence of an alkali metal hydroxide and a catalytically effective amount of a noble metal of Group VIII of the Periodic Table of the Elements, characterized in that as oxidation agent hydrogen peroxide and as noble metal palladium is used.

R in formula I preferably represents an alkyl group having of from 1 to 22, more preferably of from 8 to 20 and particularly of from 11 to 18 carbon atoms, and n is preferably an integer of from 1 to 12, more preferably of from 2 to 9. The R group can be substituted with any substituent which does not interfere with the oxidation of the hydroxy group. Such substituents include $—OR^2$, $—CH_3$, —COOH, $—CONH_2$ and $—COOR^2$, wherein $R^2$ represents an alkyl or aryl group. The $R^1$ group on an individual molecule can be hydrogen, methyl or mixtures thereof. For example, straight ethoxylates, straight propoxylates and mixed ethoxylatepropoxylate detergent alcohols are commercially available. Commercially, detergent range ethoxylate alcohols are available with an average of 3, 7, 9 and 12 ethoxylate units per molecule. Others can readily be prepared. The process is thought to proceed by the following reaction:

$$RO(CH_2CHR^1O)_nCH_2CH_2OH + NaOH + 2H_2O_2 \xrightarrow[20—100°C]{palladium} RO(CH_2CHR^1O)_nCH_2CO_2Na + 4H_2O$$

Applicant, discloses in the related patent application ·80200352.5 a mild oxidation process for the preparation of alkoxyalkanoic acids utilizing tert. butyl hydroperoxide instead of hydrogen peroxide as an oxidizing agent. Surprisingly, of the hydroperoxides, only hydrogen peroxide and tert. butyl hydroperoxide are found to be effective as oxidants. Other alkyl, aryl and acyl peroxides and their derivatives are practically ineffective.

The acid product is produced in the form of the alkali metal salt and it is to be understood that the term "acid" as used herein is intended to include the salt forms as well as the free acid form.

Although NaOH is shown in equation I as the base utilized, any hydroxide of an alkali metal is suitable: lithium, sodium, potassium, rubidium, or cesium. A strong base, such as the alkali metal hydroxide, is needed. When weaker bases such as $NaHCO_3$ were used, no reaction occurred. At least one equivalent of base for each equivalent of alcohol reacted will be necessary. It has been found that maximum conversion is obtained when a slight excess of strong base is present during the reaction. Thus, it is preferable to maintain during the reaction an excess of the alkali metal hydroxide. In other words, during the reaction, base is provided in such a manner that at least a small amount of unreacted base will be present during the course of the reaction. This requirement for a slight excess of strong base affects the manner of the base addition. As one equivalent of base is consumed per equivalent of acid product formed, the base must be added at least as fast as oxidation is proceeding in order to maintain an excess. in general it is preferred to add one equivalent of base to the starting mixture and meter the hydrogen peroxide into the reaction at slightly less than the maximum rate at which it could be consumed by the catalyst. This technique would leave no excess base in the final product. Alternately the base and hydrogen peroxide can be simultaneously metered into the reactor, with the rate of injection of hydrogen peroxide slightly less than its consumption and the base adjusted to provide a slight excess. The rates can readily be determined by routine experimentation.

The palladium catalyst used herein may be either homogeneous, heterogeneous, or a combinaton of both. Typically, palladium metal, its oxide and its salts, including organic and inorganic salts are useful as catalysts. The use of homogeneous palladium salts either above or in combination with satabilizing ligands will require a suitable means of removing the palladium ion from the product in order to make the process economic. Ion exchange resins are suitably used. Heterogeneous palladium comprises palladium metal, palladium oxide, and insoluble palladium salts, both organic and inorganic.

The catalyst utilized is preferably palladium metal, either the metal alone or supported on a suitable support. Suitable supports are those which are inert to the reaction conditions. Examples of supports suitable for certain reaction conditions are carbon, alumina, clay, silica, pumice, magnesia, zirconia and titania, etc. Some of the supports, such as alumina and silica are sensitive to base concentrations, i.e. dissolution of the support in high hydroxide concentrations. These types of supports can be used only when the excess hydroxide is low. Carbon is a highly desirable support since it is neutral to all reaction conditions. Satisfactory commercial catalysts containing up to 10 percent wt. of palladium on carbon are readily available and quite suitable for use in the process. While the unsupported catalyst is suitable, it is less preferable to the supported catalysts since more of the expensive palladium metal is required to achieve the same results as with the supported catalysts. The catalysts are frequently reduced in hydrogen prior to use to assure that the palladium is in the reduced state. Amounts of palladium to reactant alcohol are not critical, but the amount of palladium present can affect the rates. Preferably, the mole ratio of palladium to reactant alcohol is greater than 1/1000, more preferably 1/100.

The reaction is conducted under relatively mild conditions with good results being obtained using a temperature of 20°—100°C, preferably from 40° to 80°C. There are no particular pressure requirements in the process since the process is carried out in the liquid phase. Atmosphere pressure is most convenient.

The reaction may be carried out in a solvent. The preferred solvent for the system is water. Water is a coproduct of the oxidation reaction, and its presence has no effect on the oxidative process. When water is used as a solvent, it is preferably used in amounts up to about 20 percent by weight of the total amount of starting compounds. Other solvents which are inert in the reaction medium are useful, such as lower alkanes such as for example, hexane, decane, etc., lower alkanols such as ethanol, propanol, butanol, etc., and aromatics such as benzene and benzene derivatives as toluene, xylene, etc. Organic solvents are frequently used to lower the viscosity of the reacting mixture in order to allow more complete reaction to occur.

The reaction product can be purified by a number of conventional procedures. One method is acidifying the reaction product with a strong inorganic acid such as HCl to convert the product to its acid form, adding a salt such as sodium sulphate to increase the number of ions in water and thus increasing the selective solubility of the product in ether and then extracting the alkoxyalkanoic acid with ether. Next, the ether is evaporated and the acid product is dried, as by drying with a salt such as sodium sulphate or by azeotropic distillation with benzene, etc. Further purification can be effected by fractional distillation.

The yields of the alkoxyalkanoic acid obtained by this invention are excellent with, optimum conditions, conversion of the alcohol of about 70 mol %, with selectivities in excess of 95 mol% being obtained.

The process of this invention will be further described by the following illustrative examples:

In the following examples, the starting alcohol was a SHELL NEODOL® ethoxylate 23—3T alcohol which was prepared by ethoxylating a mixture of $C_{12}$ and $C_{13}$ substantially straight chain alcohols ($C_{12}$:$C_{13}$~40:60) to an ethoxylate alcohol having about three ethylene oxide units on the average per molecule and then topping off the unreacted alcohols and lower ethoxylates so that the final product has about five ethylene oxide units per molecule or a SHELL NEODOL ethoxylate 253 alcohol which was prepared by ethoxylating a mixture of $C_{12}$, $C_{13}$, $C_{14}$, and $C_{15}$ substantially straight chain alcohols ($C_{12}$:$C_{13}$:$C_{14}$:$C_{15}$~25%:40%:20%:15%) to an ethoxylate alcohol having about three ethylene oxide units on the average per molecule (ethoxylated product contains about 16 percent unethoxylated alcohol).

Conversion (basis alcohol) is calculated as the moles of alkoxyalkanoic acid divided by moles of starting alcohols times 100 (%).

Conversion (basis hydrogen peroxide) is calculated as moles of hydrogen peroxide consumed divided by moles at start times 100 (%).

Selectivity (basis alcohol) is calculated as the moles of alkoxyalkanoic acid found divided by total moles of carboxylic acid found times 100 (%).

Selectivity (basis hydrogen peroxide) is calculated as twice the moles of alkoxyalkanoic acid found divided by moles of hydrogen peroxide used times 100 (%).

### Example I

In a typical experiment a solution of NEODOL ethoxylate 23—3T, (21 g, 0.05 mol) in tert. butyl alcohol (11 ml) was first treated with NaOH (2 g, 0.05 mol). The alkaline slurry was charged with 1 g (1 mmol) of 10 wt% Pd-on-powdered-charcoal catalyst and the mixture heated to 60°C with stirring. An aqueous (30 wt%) solution of hydrogen peroxide (11 ml, 0.1 mol) was slowly (at 6 ml/h) added to the reactor. The reaction was stirred for 30 min after the final addition of oxidant. Analysis of the product found that 67 percent of the ethoxylate had been converted with a selectivity to alkoxyalkanoic acid of 98 mol%. At complete conversion of the hydrogen peroxide, 67 percent had been utilized in making alkoxyalkanoic acid.

When the reaction was repeated without the tert. butyl alcohol as solvent the reaction mixture solidified at ~51 percent conversion of the ethoxylate and further reaction was not possible. Analysis of the product, however, found the selectivity to alkoxyalkanoic acid to be 80 mol% with the utilization of the hydrogen peroxide at 84 mol%.

### Example II

A batch reactor was charged with a solution of NEODOL ethoxylate 25—3 (17.7 g, 0.051 mol), 2.2 g of NaOH (0.055 mol) and 2 g of $H_2O$ (0.11 mol). 0.15 Grams of palladium acetate (47.35 percent Pd, 0.00068 mol) was added to the reactor which was heated and stirred to 60°C. The palladium acetate appeared to dissolve in the solution but within a short time the solution turned a dark gray colour. An aqueous (30 wt%) solution of hydrogen peroxide (11 ml, 0.1 mol) was added slowly (at 6 ml/h) to the reactor. The reactor was stirred for 30 min after the final addition of the hydrogen peroxide. Analysis of the product showed that 67 percent of the ethoxylate had been converted with a selectivity to the alkoxyalkanoic acid of about 100 percent and that all of the hydrogen peroxide was converted with a selectivity of 61 percent.

## Claims

1. A process for the preparation of an alkoxyalkanoic acid of the general formula:

$$RO(CH_2CHR^1O)_nCH_2CO_2H \quad (I)$$

wherein R represents an alkyl group, optionally substituted by $-OR^2$, COOH, $CONH_2$, $COOR^2$, wherein $R^2$ represents an alkyl or aryl group, $R^1$ represents a hydrogen atom or a methyl group or — on the individual molecule — a mixture thereof and n is an integer, by reacting an alkoxyalkanol of the general formula:

$$RO(CH_2CHR^1O)_nCH_2CH_2OH \quad (II)$$

wherein R, $R^1$ and n have the same meaning as in formula I, in the liquid phase with an oxidation agent in the presence of an alkali metal hydroxide and a catalytically effective amount of a noble metal of Group VIII of the Periodic Table of the Elements, characterized in that as oxidation agent hydrogen peroxide and as noble metal palladium is used.

2. A process as claimed in claim 1, characterized in that R in formula I represents an alkyl group having of from 1 to 22 carbon atoms and n is an integer of from 1 to 12.

3. A process as claimed in claim 2, characterized in that R in formula I represents an alkyl group having of from 11 to 18 carbon atoms and n is an integer of from 2 to 9.

4. A process as claimed in any one of the preceding claims, characterized in that the reaction is carried out at a temperature in the range of from 20° to 100°C.

5. A process as claimed in any one of the preceding claims, characterized in that during the course of the reaction a small amount of

unreacted alkali metal hydroxide is present.

6. A process as claimed in claim 1 characterized in that the palladium is homogeneous palladium.

7. A process as claimed in claim 1 characterized in that the palladium is heterogeneous palladium.

8. A process as claimed in claim 7 characterized in that the heterogeneous palladium is palladium metal supported on carbon.

**Revendications**

1. Un procédé pour la préparation d'un acide alcoxyalcanoïque de la formule générale:

$$RO(CH_2CHR^1O)_nCH_2CO_2H \quad (I)$$

dans laquelle R représente un groupe alcoyle, éventuellement substitué par —$OR^2$, COOH, $CONH_2$, $COOR^2$, où $R^2$ représente un groupe alcoyle ou aryle, $R^1$ représente un atome d'hydrogène ou un groupe méthyle ou, pour la molécule individuelle, un mélange d'atomes d'hydrogène et de groupes méthyle et n est un nombre entier, en faisant réagir un alcoxyalcanol de la formule générale:

$$RO(CH_2CHR^1O)_nCH_2CH_2OH \quad (II)$$

dans laquelle R, $R^1$ et n ont la même signification que dans la formule I, dans la phase liquide avec un agent d'oxydation en présence d'un hydroxyde de métal alcalin et d'une quantité catalytiquement efficace d'un métal noble du groupe VIII de la table périodique des éléments, caractérisé en ce que comme agent d'oxydation on utilise de l'eau oxygénée et comme métal noble on utilise le palladium.

2. Un procédé selon la revendication 1, caractérisé en ce que R dans la formule I représente un groupe alcoyle ayant de 1 à 22 atomes de carbone et n est un nombre entier de 1 à 12.

3. Un procédé selon la revendication 2, caractérisé en ce que R dans la formule I représente un groupe alcoyle ayant de 11 à 18 atomes de carbone et n est un nombre entier de 2 à 9.

4. Un procédé selon l'une quelconque des revendications précédentes, caractérisé en ce que la réaction est conduite à une température comprise entre 20°C et 100°C.

5. Un procédé selon l'une quelconque des revendications précédentes, caractérisé en ce que durant le cours de la réaction une petite quantité d'hydroxyde de métal alcalin n'ayant pas réagi est présente.

6. Un procédé selon la revendication 1, caractérisé en ce que le palladium est du palladium homogène.

7. Un procédé selon la revendication 1, caractérisé en ce que le palladium est du palladium hétérogène.

8. Un procédé selon la revendication 7, caractérisé en ce que le palladium hétérogène est du palladium métallique déposé sur du carbone.

**Patentansprüche**

1. Ein Verfahren zur Herstellung einer Alkoxyalkancarbonsäure der allgemeinen Formel

$$RO(CH_2CHR^1O)_nCH_2CO_2H \quad (I)$$

in der R eine Alkylgruppe darstellt, die gegebenenfalls mit —$OR^2$, COOH, $CONH_2$ $COOR^2$ substituiert ist, wobei $R^2$ eine Alkyl- oder Arylgruppe darstellt, $R^1$ ein Wasserstoffatom oder eine Methylgruppe darstellt oder — am einzelnen Molekül — ein Gemisch der beiden, und n eine ganze Zahl ist, durch Umsetzen eines Alkoxyalkanols der allgemeinen Formel:

$$RO(CH_2CHR^1O)_nCH_2CH_2OH \quad (II)$$

in der R, $R^1$ und n die gleiche Bedeutung haben wie in Formel I, in flüssiger Phase mit einem Oxidationsmittel in Gegenwart eines Alkalimetallhydroxids und einer katalytischwirksamen Menge eines Edelmetalls der Gruppe VIII des Periodensystems der Elemente, dadurch gekennzeichnet, daß als Oxidationsmittel Wasserstoffperoxid und als Edelmetall Palladium verwendet wird.

2. Ein Verfahren wie in Anspruch 1 beansprucht, dadurch gekennzeichnet, daß R in Formel I eine Alkylgruppe mit 1 bis 22 Kohlenstoffatomen darstellt und n eine ganze Zahl von 1 bis 12 ist.

3. Ein Verfahren wie in Anspruch 2 beansprucht, dadurch gekennzeichnet, daß R in Formel I eine Alkylgruppe mit 11 bis 18 Kohlenstoffatomen darstellt und n eine ganze Zahl von 2 bis 9 ist.

4. Ein Verfahren wie in einem der vorstehenden Ansprüche beansprucht, dadurch gekennzeichnet, daß die Reaktion bei einer Temperatur im Bereich von 20 bis 100°C durchgeführt wird.

5. Ein Verfahren wie in einem der vorstehenden Ansprüche beansprucht, dadurch gekennzeichnet, daß im Verlauf der Reaktion eine geringe Menge von nicht umgesetztem Alkalimetallhydroxid vorhanden ist.

6. Ein Verfahren wie in Anspruch 1 beansprucht, dadurch gekennzeichnet, daß das Palladium homogenes Palladium ist.

7. Ein Verfahren wie in Anspruch 1 beansprucht, dadurch gekennzeichnet, daß das Palladium heterogenes Palladium ist.

8. Ein Verfahren wie in Anspruch 7 beansprucht, dadurch gekennzeichnet, daß das heterogene Palladium Palladiummetall auf einem Kohlenstoffträger ist.